Europäisches Patentamt

**(19)** European Patent Office

Office européen des brevets

**(11)** **EP 0 650 366 B1**

**(12)** **EUROPEAN PATENT SPECIFICATION**

**(45)** Date of publication and mention
of the grant of the patent:
**24.11.1999 Bulletin 1999/47**

**(21)** Application number: **92919722.6**

**(22)** Date of filing: **28.08.1992**

**(51)** Int. Cl.[6]: **A61K 38/00**, A61K 38/28,
A61K 38/27

**(86)** International application number:
**PCT/US92/07341**

**(87)** International publication number:
**WO 93/04691 (18.03.1993 Gazette 1993/08)**

**(54) COMPOSITIONS AND METHODS FOR TREATING WOUNDS**

ZUSAMMENSETZUNGEN UND METHODEN ZUR WUNDBEHANDLUNG

COMPOSITION ET PROCEDE DE TRAITEMENT DE BLESSURES

**(84)** Designated Contracting States:
**AT BE CH DE DK ES FR GB GR IE IT LI LU MC NL SE**

**(30)** Priority: **30.08.1991 US 752849**

**(43)** Date of publication of application:
**03.05.1995 Bulletin 1995/18**

**(73)** Proprietor:
**Technion Research & Development Foundation Ltd.**
**Haifa 32000 (IL)**

**(72)** Inventor: **LINDENBAUM, Ella**
**35 646 Haifa (IL)**

**(74)** Representative:
**Lally, William et al**
**FORRESTER & BOEHMERT**
**Franz-Joseph-Strasse 38**
**80801 München (DE)**

**(56)** References cited:
**EP-A- 0 364 266          EP-A- 0 516 901**
**WO-A-90/03810           US-A- 4 673 649**

- **PROCEEDINGS OF THE NATIONAL ACADEMY OF SCIENCES OF USA, vol.84, no.21, November 1987, WASHINGTON US pages 7696 - 7700 LYNCH S.E. ET AL. 'Role of platelet-derived growth factor in wound healing: Synergistic effects with other growth factors'**
- **THE JOURNAL OF CELL BIOLOGY, vol.109, July 1989 pages 429 - 440 PIERCE G.F. ET AL. 'Platelet-derived Growth Factor and Transforming Growth Factor Enhance Tissue Repair Activities by Unique Mechanisms'**
- **Journal of Cellular Physiology, Volume 121, issued 1984, JOHN L. WILLE Jr. et al., "Integrated Control of Growth and Differentiation of Normal Human Prokeratinocytes Cultured in Serum-Free Medium: Clonal Analyses, Growth Kinetics, and Cell Cycle Studies", pages 31-34, see the entire document, particularly the Abstract.**

**Description**

FIELD OF THE INVENTION

The present invention relates to novel compositions and methods using such compositions for promoting wound healing of skin and related tissues. More particularly, the invention relates to wound healing compositions which are based on a cellular nutrient medium in combination with at least one anabolic protein growth hormone or growth factor.

BACKGROUND OF THE INVENTION

A skin wound is defined as a breach in the continuity of any body tissue caused by a minimal direct injury to the skin. There are many instances where a quick closure of the wounded skin will promote a beneficial response. Generally, quick closure of wounded skin can be achieved either by conservative methods such as the application of medicaments, or alternatively, by using various surgical procedures including suturing, split skin grafting or grafting of new skin grown in culture.

The closure of a wound with skin cells is performed using two methods: either by grafting skin grown in culture or alternatively, by split skin grafting. These two methods are applicable, however, only after a suitable base of granulation tissue has first developed in the wound, the development of which may be quite prolonged or complicated. Split skin grafting, although more common, requires compositions which contain materials for maintaining organ viability and treatment of the wounds for the repair of injury to the skin.

Among the most common injuries to skin are burns. Burn causes destruction of the epidermis and deeper cutaneous and subcutaneous tissues, most of which can be regenerated by the normal healing response if the area burned is not extensive or contaminated. Burns cause more than 2,000,000 injuries annually in the U.S.A., and more than 10,000 deaths each year result from serious burn injuries.

S.T. Boyce et al., in The Journal of Investigative Dermatology, 81: 33S-40S, 1983) describes compositions based upon a serum-free culture system to culture normal human epidermal keratinocytes. These compositions comprise optimized nutrient medium MCDB 153 supplemented with epidermal growth factor, insulin, hydrocortisone, ethanolamine, phosphoethanolamine and whole Bovine Pituitary Extract (wBPE). It is mentioned that the wBPE initiates the primary culture and that cellular senescence occurs after about forty population doublings. It has also been reported in the Journal of Cellular Physiology, 110, 219, (1982), that the incorporation of Fetal Bovine Serum Protein (FBSP) may replace whole serum for culturing human epidermal keratinocytes and that the presence of F12 would eliminate the need for wBPE. As presently known, wBPE is not a common reagent which can be easily reproducibly prepared, its constitution not being constant.

J.J. Wille, Jr. et al., in the Journal of Cellular Physiology, 121, 31, (1984) describes the effects of growth factors, hormones and calcium on the growth and differentiation of secondary cultures of normal human prokeratinocytes. Clonal growth was achieved when MCBD 153 was supplemented with epidermal growth factor or wBPE, provided that insulin was present. In the absence of insulin both EGF and wBPE are required. It is mentioned that optimal clonal growth occurred in medium containing 10 ng/ml of epidermal growth factor and 0.3 mM calcium.

According to U.S. Patent No. 4,673,649, compositions are suggested for clonal growth of a population of human keratinocyte cells in a primary culture for the repair of injury to skin, having a characteristic colony-forming efficiency of about 20%. The composition comprises: MCDB 153, epidermal growth factor a concentration range of 1.0 ng/ml to 25 ng/ml and insulin at a concentration range of 0.5 ug/ml to 50 ug/ml. Optionally, the compositions may contain wBPE (whole bovine pituitary extract) at a concentration range of 7 ug/ml to 700 ug/ml, ethanolamine, hydrocortisone, phosphoethanolamine and calcium chloride. In particular, the compositions are useful for growing skin cells for grafting. No mention is made to the possible use of the disclosed compositions to treat wound conditions in vivo, nor to prolong and preserve the viability of stored split skin grafts. In a very recent U.S. Patent No. 4,940,666 (by the same inventors and as a c.i.p. of the previous U.S. Patent), the same compositions are claimed to be useful for growing a population of human epidermal cells. The purpose of the compositions suggested is for the propagation of skin cells and achieving monolayers, or stratified layers, of keratinocytes to be used for areas on the body without skin. In other words, these compositions are used for the development of cultured skin cells which may be used for grafting. In addition to the above references, other prior art references suggest that epidermal growth factor may enhance wound healing by increasing fibroblast proliferation.

OBJECTS OF THE PRESENT INVENTION

It is an object of the present invention to provide novel compositions useful for accelerating wound healing.

It is another object of the present invention to provide novel compositions which accelerate wound healing and which also prolong the viability of the skin and other tissues.

It is yet another object of the present invention to provide novel compositions useful for accelerating wound healing which comprise defined and readily recognized constituents.

It is still a further object of the present invention to provide wound healing compositions which accelerate wound healing by maintaining moisture at the wound surface.

It is still a further object of the present invention to provide wound healing compositions which maintain moisture at the wound surface and promote wound healing through use of hydrated hydrogel polymer delivery systems.

These and other objects of the present invention may be readily gleaned from the description of the present invention presented hereinbelow.

BRIEF DESCRIPTION OF THE INVENTION

The present invention relates to formulations which are useful for treating wounds by accelerating wound healing. These formulations comprise an effective amount of a cellular nutrient medium, preferably a serum free cellular nutrient medium, in combination with an effective amount of at least one cellular growth stimulating compound, e.g. a non-steroidal, preferably natural anabolic hormone or transforming growth factor.

Thus, according to the invention, there is provided a formulation for use in the vivo treatment of wounds in animals and humans comprising wound healing effective amounts of a non-steroidal anabolic hormone selected from the group consisting of insulin, tri-iodothyronine, thyroxine and mixtures thereof, at a concentration of at least about 0.05 ng/ml in a cellular nutrient medium.

In preferred embodiments according to the present invention, the formulations include growth hormone and most preferably human growth hormone as at least one of the cellular growth stimulating compounds. In compositions which are delivered in solid or concentrated form, i.e. as a gel, creme, elixir, powder or the like, the cellular growth stimulating compound is included in concentrations similar to those contained in the solutions, and preferably comprises about 0.00000005% to about 0.000005% by weight of the wound treating composition.

The amount and type of cellular growth stimulating compound may vary, but the preferred compound is human growth hormone, most preferably in combination with insulin and/or triiodothyronine (T3). The preferred amount of human growth hormone to be used will generally depend on the type and size of the wound, but generally and in most of the cases the amount of growth hormone used will be in the range of between about 0.5 ng/ml to about 50 ng/ml by weight or more of the composition. In the case of compositions which are delivered in solid or concentrated form as a gel, cream, elixir, powder or the like, human growth hormone is included in an amount ranging from about 0.5 ng/ml to about 50 ng/ml by weight or more (about 0.00000005% to about 0.000005% by weight of the wound treatment composition).

BRIEF DESCRIPTION OF THE FIGURES

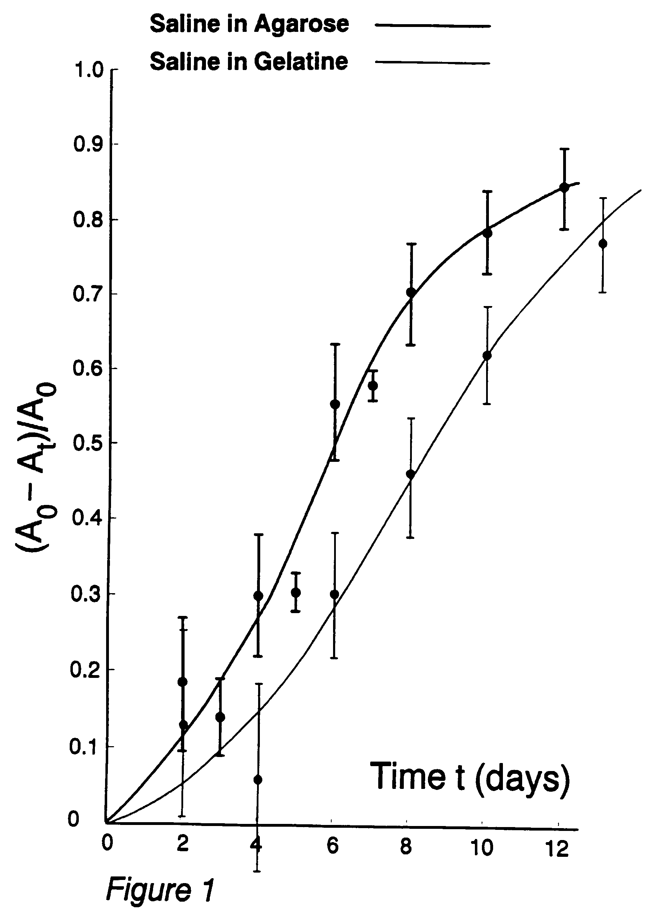

Figures 1-6 represent the results Of the experiments performed and described in Examples 5-11. These graphs show the fractional change in area of wounds treated with gel-media + hormones vs. various controls. $A_o$ represents the initial wound area and $A_t$ represents the wound area at day t.

DETAILED DESCRIPTION OF THE INVENTION

In describing the present invention in the specification, a number of terms will be used.

The term "wound" is used throughout the specification to describe skin wounds which are treated by the formulations and the method according to the present invention. A skin wound is defined herein as a breach in the continuity of skin tissue which is caused by direct injury to the skin. Skin wounds are generally characterized by several classes: punctures, incisions, including those produced by a variety of surgical procedures, excisions, lacerations, abrasions and burns, including large burn areas. The formulations according to the present invention are useful in varying degrees for enhancing the healing of all wounds of the skin, including those which occur after a mesh autograph procedure.

The term "delivery polymer" is used throughout the specification to describe a polymer which can be used in combination with a cellular nutrient medium (preferably, serum free) and a cell growth stimulating compound to produce formulations which are preferably used for topical administration to treat wounds according to the present invention. These delivery polymers include, for example, numerous hydrogels in hydrated or unhydrated form, such as hydroxyethylmethacrylate (HEMA), glycerolmethacrylate (GMA) and polyvinylpyrrolidone (PVP), polyethylene glycol (PEG), collagen, gelatin, agarose (for example, as an agarose saturated gel), related polymers and mixtures thereof. One of ordinary skill in the art will recognize to vary the type and amount of delivery polymer in compositions according to the present invention to provide enhanced wound healing characteristics appropriate for topical delivery. The term delivery polymer is also used to describe polymers which instill slow-release or sustained release characteristics to the wound healing formulations of the invention.

The term "serum free cellular nutrient medium" is used throughout the specification to describe a medium which contains no serum, and in combination with a cell growth stimulating compound comprises wound healing compositions according to the present invention. The serum free nutrient medium according to the present invention comprises the following elements: (a) essential amino acids; (b) non-essential amino acids; and (c) vitamins selected from the group consisting of biotin, folate, lipoate, niacinamide, pantothenate, pyridoxine, riboflavin, thiamin and vitamin $B_{12}$. All of these elements (a), (b) and (c) are included with the cell growth stimulating compounds in concentrations and/or amounts effective for enhancing the growth of cells which surround, have been injured by or are responsible for healing a wound. The preferred concentration of essential and non-essential amino acids used in the present invention ranges from about 5.0 um ($10^{-6}$ mole) to about 50 mmol. ($10^{-3}$ mole) The preferred concentrations of vitamins used in the present invention ranges from about 1 nanomole ($10^{-9}$ mol.) to about 10 um. In addition to the elements (a), (b) and (c), the nutrient medium according to the present invention optionally contains any one or more of the following elements: (d) purines and pyrimidines; (e) other organic compounds; (f) major inorganic ions; (g) trace elements; (h) buffers and indicators and (i) other supplements. All of the elements (d), (e), (f), (g), (h) and (i), where they are included in the nutrient medium according to the present invention, are included in amounts effective for enhancing the growth of cells involved in the wound-healing processes. Preferably, components (d), (e), (h) and (i) range in concentration from about 1 nmol. to about 10 mmol. In the case of components (f) and (h), the concentration preferably ranges from about 1 umol. to about 50 mmol. One of ordinary skill in the art will be able to readily modify the type and amount of the components of the cellular nutrient medium within the teaching of the present invention.

In addition to serum free cellular nutrient medium, the present invention may also make use of cellular nutrient medium containing serum, although the use of a serum containing cellular nutrient medium is generally less preferred than is serum free medium. Examples of such nutrient medium include, among numerous others, DMEM, HAM F12 and HAM F10, all containing serum. The term "cellular nutrient medium" is used to describe all types of nutrient medium contemplated for use in the present invention, including serum free cellular nutrient medium.

The cellular nutrient medium according to the present invention may include commercially available media in solution or lyophilate form. The cellular nutrient medium used may be in the form of a lyophilate which may be reconstituted with water, preferably sterilized, distilled water and then supplemented with a cell growth stimulating compound or other additives. Alternatively, the nutrient medium may be used directly in formulations according to the present invention in the form of a lyophilate, or related solid-type material, rather than a solution, especially when gels, creams, elixirs, powders or other delivery vehicles are to be used for delivery. It is clearly preferred when utilizing solid-type materials for delivering the wound healing compositions according to the present invention that the delivery system in the form of a hydrogel or other form contain moistening quantities of water.

Many of the commercially available media (preferably, serum free) are available from suppliers such as Collaborative Research Incorporated, Bedford Massachusetts or Biological Industries, Beth HaEmek, Israel. These media may be used as purchased or modified within the scope and practice of the present invention.

The term "cell growth stimulating compound" or "cellular growth stimulating compound" is used throughout the specification to describe those compounds which are added to the formulations according to the present invention for their known benefits in stimulating the growth and elaboration of cells. Cell growth stimulating compounds for use in the present invention include non-steroidal anabolic hormones, for example, thyroxine ($T_4$), triiodothyronine ($T_3$), insulin, and mixtures thereof. The formulations may however also include human growth hormone, and growth factors, such as insulin-like growth factor (IGF). In the formulations according to the present invention, one or more cell growth stimulating compound is included in an amount effective for stimulating the growth of cells which surround, have been injured by, or are responsible for healing a wound. Cell growth stimulating compounds for use in the present invention may include naturally isolated or synthetically produced versions of the abovemetioned compounds or their equivalents and include, where relevant, compounds produced by genetic engineering processes and techniques.

The amount of each component which is used in the formulations according to the present invention will depend upon the type and size of the wound, but each component is included in an amount effective for significantly enhancing the healing of a wound relative to traditional wound healing therapies. In general, in preferred embodiments according to the present invention, the formulations include a cell growth stimulating compound at a concentration of at least about 0.05 ng/ml, preferably about 0.5 ng/ml to about 50 ng/ml or more. In the case of formulations containing insulin, the amount of insulin often falls outside this range. Preferably, the cell growth stimulating compound is human growth hormone and/or insulin, because of their known benefits in promoting the growth and elaboration of cells and their general absence of toxicity.

The preferred human growth hormone is a well-known defined protein which is readily available and results from a pituitary secretion into the blood system. It is constituted from a number of amino acids with a total molecular weight of about 193,000. The human growth hormone which may be used in the present invention can be obtained from a variety of sources, including genetic engineering processes and techniques.

A particularly preferred cell growth stimulating compound for use in the present invention comprises a mixture of an effective amount of human growth hormone, optionally in the presence of an effective amount of insulin (transferrin containing or transferrin-free) and/or triiodothyronine ($T_3$) or thyroxine ($T_4$), preferably in a serum free cellular nutrient medium. In this preferred embodiment of the instant invention, each cell growth stimulating compound is included in the final composition in an amount ranging from about 0.05 to about 50 ng/ml or higher concentration, and preferably about 1 ng/ml to about 20 ng/ml or more of the final composition. The amount of insulin is preferably included in amounts ranging from about 5ng/ml to about 100ug/ml (about 0.1 mUnits/ml to about 2 U/ml), preferably about 50 mg/ml to about 2ug/ml. One of ordinary skill in the art will know to vary the amount of cellular growth stimulating compound within effective ranges based upon the type and potency of the preparation of the compound.

The cellular nutrient medium which is used in the present invention is any nutrient medium having the effect of enhancing recovery of wounded skin tissue when used in combination with the cell growth stimulating compound. In preferred embodiments according to the present invention, the cell growth stimulating compound in an effective amount is mixed into serum free cellular nutrient medium to form the compositions according to the present invention.

The cellular nutrient medium comprises the following groups of constituents: (a) essential amino acids; (b) non-essential amino acids; (c) vitamins; (d) purines and pyrimidines; (e) other organic compounds; (f) major inorganic ions; (g) trace elements; (h) buffers and indicators and (i) other supplements. The groups (d), (e), (f), (g), (h) and (i) are optional. Serum free cellular nutrient medium is preferred. The preferred serum free cellular nutrient medium is modified MCDB.

While not being limited by way of theory, it is believed that one plausible explanation of the mechanism of the accelerated wound healing is that the presence of the cellular growth stimulating hormone, and in particular, human growth hormone or insulin in the formulations according to the present invention, promotes the growth in situ of the granulation tissue, i.e., within the wound itself. At the same time, the novel formulations may also induce the stimulation of the vascular elements and promote the growth of vascularized granulation tissue preparatory to split skin grafting. The proliferation of vascularized granulation promotes epidermal growth from the peripheral edges of the wound over the vascular substratum and from deeper layers of the dermis leading to an early closure of the skin over the wound. The mechanism which might be assumed is that during the proliferation phase, new capillaries and fibroblasts appear in the wound from the first day on and reach their maximum levels after one week. The new vessels in granulation tissue originate as budlike structures on nearby vessels, penetrate the wound, become canalized and ramify throughout the wound by cellular division.

It is further believed that the function of the nutrient medium is to provide nutrients to normal, distressed and injured cells which surround or comprise the wound to be treated in order to enhance the growth and repair mechanisms which are responsible for the healing of the wound. In this way, the nutrient medium functions to enhance the ability of the cellular growth stimulating hormone to promote the elaboration, growth and healing of the wound. In addition, the media serves to maintain a moist environment surrounding the wound area.

A number of cellular nutrient media, preferably serum free, may be used in the present invention, including commercially available media or other media well known in the art. Examples of such media (all without serum or having had the serum removed) include ADC-1, LPM (Bovine Serum Albumin-free), F10 (HAM), F12 (HAM), DCCM1, DCCM2, RPMI 1640, BGJ Medium (Fitton-Jackson Modification), Basal Medium Eagle (BME-with the addition of Earle's salt base), Dulbecco's Modified Eagle Medium (DMEM-without serum), Glasgow Modification Eagle Medium (GMEM), Leibovitz L-15 Medium, McCoy's 5A Medium, Medium M199 (M199E- with Earle's salt base), Medium M199 (M199H- with Hank's salt base), Minimum Essential Medium Eagle (MEM-E- with Earle's salt base), Minimum Essential Medium Eagle (MEM-H-with Hank's salt base) and Minimum Essential medium Eagle (MEM-NAA- with non-essential amino acids), among numerous others. These and other useful serum free cellular nutrient media are available from Biological Industries, Bet HaEmek, Israel.

In addition, serum-containing cellular nutrient media may also be used in compositions according to the present invention, but the use of serum-containing media is less preferred because of the possibility that the serum may be contaminated with microbial agents and because the patient may develop immunological reactions to certain antigenic components contained in the serum.

While a large number of serum free cellular nutrient media may be used in the present invention, a preferred nutrient media for use in the present invention is modified MCDB 153, a most preferred serum free cellular nutrient medium.

Experiments which were carried out to prolong the viability of human split graft specimens show that the use of the modified MCDB 153 medium according to the present invention, extended the limit of viability from 3 to 9 weeks. Histological examination of the split skin specimens indicated a strong attachment of the epidermal layer to the dermal substratum in all specimens kept in the modified MCDB 153 medium at 20°C.

Hereafter are enumerated the particular constituents and concentrations of the above groups for MCDB 153:

| Group (a): | Concentration |
|---|---|
| Arginine | $1.0 \times 10^{-3}$ |
| Cysteine | $2.4 \times 10^{-4}$ |
| Glutamine | $6.0 \times 10^{-3}$ |
| Histidine | $8.0 \times 10^{-5}$ |
| Isoleucine | $1.5 \times 10^{-5}$ |
| Leucine | $5.0 \times 10^{-4}$ |

| | |
|---|---|
| Lysine | $1.0 \times 10^{-4}$ |
| Methionine | $3.0 \times 10^{-5}$ |
| Phenylalanine | $3.0 \times 10^{-5}$ |
| Threonine | $1.0 \times 10^{-4}$ |
| Tryptophan | $1.5 \times 10^{-5}$ |
| Tyrosine | $1.5 \times 10^{-5}$ |
| Valine | $3.0 \times 10^{-4}$ |
| | |
| Group (b): | |
| Alanine | $1.0 \times 10^{-4}$ |
| Asparagine | $1.0 \times 10^{-4}$ |
| Aspartate | $3.0 \times 10^{-4}$ |
| Glutamate | $1.0 \times 10^{-4}$ |
| Glycine | $1.0 \times 10^{-4}$ |
| Proline | $3.0 \times 10^{-4}$ |
| Serine | $6.0 \times 10^{-4}$ |
| | |
| Group (c): | |
| Biotin | $6.0 \times 10^{-8}$ |
| Folate | $1.8 \times 10^{-6}$ |
| Lipoate | $1.0 \times 10^{-6}$ |
| Niacinamide | $3.0 \times 10^{-7}$ |
| Pantothenate | $1.0 \times 10^{-6}$ |
| Pyridoxine | $3.0 \times 10^{-7}$ |
| Riboflavin | $1.0 \times 10^{-7}$ |
| Thiamin | $1.0 \times 10^{-6}$ |
| Vitamin B12 | $3.0 \times 10^{-7}$ |
| | |
| Group (d): | |
| Adenine | $1.8 \times 10^{-4}$ |
| Thymidine | $3.0 \times 10^{-6}$ |
| | |
| Group (e): | |
| Acetate | $3.7 \times 10^{-3}$ |
| Choline | $1.0 \times 10^{-4}$ |
| Glucose | $6.0 \times 10^{-3}$ |
| i-Inositol | $1.0 \times 10^{-4}$ |
| Putrescine | $1.0 \times 10^{-6}$ |
| Pyruvate | $5.0 \times 10^{-4}$ |

Group (f):

| | |
|---|---|
| Magnesium | $6.0 \times 10^{-4}$ |
| Postassium | $1.5 \times 10^{-3}$ |
| Sodium | $1.5 \times 10^{-1}$ |
| Chloride | $1.3 \times 10^{-1}$ |
| Phosphate | $2.0 \times 10^{-3}$ |
| Sulfate | $4.5 \times 10^{-6}$ |

Group (g):

| | |
|---|---|
| Copper | $1.0 \times 10^{-8}$ |
| Iron | $1.5 \times 10^{-6}$ |
| Zinc | $3.0 \times 10^{-6}$ |

Group (h):

| | |
|---|---|
| Bicarbonate | $1.4 \times 10^{-2}$ |
| HEPES | $2.8 \times 10^{-2}$ |

Group (i):

| | |
|---|---|
| Ethanolamine | 0.1 mmol. |
| Phosphoethanolamine | 0.1 mmol. |
| Calcium | 0.1 mmol. |

Weights of each of the above components in the medium may be varied within the concentrations described hereinabove to provide formulations workable within the description of the present invention.

Preferably, the cell growth stimulating compound to be incorporated into the modified MCDB 153 composition, according to the present invention, is human growth hormone in an effective amount of at least about 0.05 ng/ml. Most preferably, the cell growth stimulating compound includes a mixture of human growth hormone, insulin (containing transferrin or transferrin-free) and/or triiodothyronine ($T_3$) or thyroxin ($T_4$), each compound in an amount ranging from at least about 0.05ng/ml, preferably at least about 0.5ng/ml, and more preferably at least about 1 ng/ml or more. In the case of insulin, the effective amount of insulin generally ranges from about 5ng/ml to about 100ug/ml and more preferably about 50 ng/ml to about 2ug/ml within this range.

In addition to effective amounts of cellular growth stimulating hormone and cellular nutrient media, formulations according to the present invention may also contain hydrocortisone, which in certain instances may have a beneficial overall effect in enhancing wound healing.

Hydrocortisone is found to improve the cloning efficiency of fibroblasts, enhancing the maintenance of epidermal keratinocytes. The preferred amount to be incorporated is generally within the range of about 0.2 umol. to about 50 umol.

Insulin/transferrin is an optional and desirable constituent cell growth stimulating compound, found to impart a maturing stimulus of the growing culture. The preferred amount of insulin is in the range of about 5 ng/ml to about 100ug/ml (about 0.1 mUnits/ml to about 2U/ml) and more preferably about 50 ng/ml to about 2ug/ml within this range. Insulin is preferably included in wound-treatment formulations according to the present invention along with at least one other growth stimulating compound. Insulin may be commercially obtained and is generally provided in mU quantities (about 41 ng of insulin). The International Unit of Insulin (SI= System International) is the activity contained in 0.04167 mg (41.67 ug) of the 4th International Standard Preparation (1958). The Standard Preparation is a quantity of purified Zinc Insulin crystals extracted 52% from Bovine and 48% from Porcine pancreas (See, Martindale Pharmacopoeia, 26th Ed.).

The formulations according to the present invention may also include an effective amount of an antimicrobial agent, for example, antibiotics and antifungal agents, such as griseofulvin and nystatin and antiviral agents and the like. The antimicrobial agent may be added for its ability to treat an infection, or alternatively, for its prophylactic effect in avoiding an infection. Where antimicrobial agents are contemplated for use in the present invention, an amount effective to treat an infection or a prophylactic amount of such agent is chosen. The amount of antimicrobial agent used is that amount typically used in topical applications. One of ordinary skill in the art can easily determine the type and amount of antimicrobial agent chosen for use in formulations according to the present invention. In general, the amount of antimicrobial agent may vary widely according to the efficacy of the agent to be delivered and the prophylactic treatment or the severity of the infection. However, in general, the amount of antimicrobial agent to be used in the present invention will range from about .05 ug/ml to about 250 mg/ml with a preferred range of about 50 to about 200 ug/ml. Of course, these ranges will vary depending upon the condition of the infection to be treated as well as the strength of the antimicrobial agent employed. For example, in the case of treatment of fungal infections, the amount of amphotericin used generally ranges from about 0.1 ug/ml to about 100 ug/ml with a preferred concentration of about 0.25 ug/ml. In the case of antibiotics and in particular, penicillin, streptomycin and gentamycin, these agents are generally utilized within the concentration range of about .05 ug/ml to about 250 mg/ml. with a preferred concentration range of about 25 ug/ml to about 250 ug/ml.

In the case of the use of antibiotics, any number of antibiotics may be used, including aminoglycosides, sulfa drugs, penicillins and chloramphenicol, among others, but it is preferable to use the broad spectrum antibiotics, for example, a cephalosporin or tetracycline in a prophylactic amount or alternatively, in an amount effective for treating a bacterial infection. In using antibiotics, one of ordinary skill in the art will recognize to minimize or avoid the use of antibiotics which may produce allergic reactions in the treated patients.

In certain embodiments according to the present invention, the formulations as described herein are further formulated with hydrogels or related delivery polymers for delivering the formulations according to the present invention to the wound. In these embodiments, the formulations comprising effective amounts of cellular growth stimulating hormone and serum free cellular nutrient media, either alone or in addition to other components, are admixed with a delivery polymer, for example a hydrogel such as HEMA (hydroxyethylmethacrylate) or NVP (N-vinylpyrrolidone), polyethylene glycol (PEG), gelatin, agarose, methylcellulose and related hydrophilic cellulose polymers or collagen to promote wound healing. In addition to accelerating wound healing through application of the formulations of the present invention, the compostions which are formulated with a delivery polymer also exhibit the added benefit of preventing or slowing the formation of a scab on the wound. While not being limited by way of theory, it is believed that the resultant wound tissue, which remains soft and moist instead of dry and scab-like, produces a beneficial, cosmetically pleasing and increased rate wound-healing.

In addition to solution, gel or hydrogel forms, compositions according to the present invention also may be formulated as creams, elixirs, powders and the like.

In a method for treating wounds according to the present invention, the formulations as described hereinabove are topically applied to the wound tissue as a liquid or gel at least once a day and up to six or more times a day. In the case of formulations containing a delivery polymer, the formulations may be administered less frequently than when the formulations are applied as a liquid. One of ordinary skill in the art will readily determine the amount and frequency of administering the formulations according to the present invention. The amount of material which is to be spread on a wound for treatment will be readily apparent to one of ordinary skill in the art. In general, in solution or gel form, about 1 cc of formulation is applied per $cm^2$ to the wound area. Depending upon the depth of the wound to be treated, an amount greater or less than 1 cc of formulation per $cm^2$ of the wound surface may be utilized. In many instances, the depth of the formulation on the wound should be at least about 2 mm.

Preliminary bioassays to determine the acceleration of wound healing which were carried out on rats, guinea pigs and on selected clinical cases indicated that the formulations according to the present invention exhibited a significant beneficial result relative to traditional therapies.

The invention will be described hereinafter by a number of Examples which illustrate some actual tests carried out on wounds treated with the compositions according to the present invention. It should be understood that the Examples are not exhaustive nor limiting and are presented only for a better understanding of the invention.

## EXAMPLES EXAMPLE 1

Wound-Healing Formulation

100 g. of Lyophilized powder of MCDB 153 was reconstituted with distilled, sterilized $H_2O$ and supplemented with human growth hormone to a final concentration of about 0.5 to about 2 ng/ml by conventional mixing. In certain formulations, an amount of insulin-transferrin was added to a final concentration of about 5 mUnits/ml (about 200ng/ml). The resulting solution was used to treat wounds as exemplified by the following wound-treatment examples. In certain

EP 0 650 366 B1

instances, about 1% by weight gelatin or collagen was added to provide a gel product for delivery to wounds as indicated.

## EXAMPLE 2

Heel Decubitus-pressure wound.

A woman suffering from an acute Toxic Epidemolysis Necrosis (TEN), due to hypersensitivity to sulfa medication developed an oval shape pressure wound (10x5x2cm) on her right heel. Conservative treatment failed to produce a successful result.

First treatment consisted of the application of a liquid composition of the formulation according to the present invention containing 1.0 ng/ml of human growth hormone and covered by a bandage.

Three days later, the bandage, stained with exudate which had seeped through, was removed. Proliferation of granulation tissue was noticed in the wound bed. The initial oval-shaped contour of the boundary was now keyhole-shaped, having a reduced size of 7x3x1 cm. A similar treatment with the same composition as above was applied on the wound.

Three days later, the bandage was found to be dried and was removed. The wound appeared to be substantially narrowed and had a size of 5x1.5x0.5 cm. The granulation tissue in the wound was highly vascularized. A similar treatment with the same composition as above was applied. Three days later the dry bandage was removed and the wound was found to be completely closed.

## EXAMPLE 3

Old chronic leg wound

A 16 month old chronic tapered-oval-shaped crural ulcer (7x3.5x2 cm) was located on the anterior aspect of the upper third tibia. A conventional treatment which was applied was repeatedly unsuccessful.

A collagen gel of the composition according to Example 1 containing 0.5 ng/ml of growth human hormone was applied and covered by a bandage.

Three days later, the exudate-stained bandage was removed. Granulation tissue and vascularization were clearly noticed in the wound bed. The size of the wound was found to be 5.5x2.5x1 cm and its contour was rounded-oval-shaped. A second treatment with the same collagen gel of the modified MCDB 153 composition as above was applied.

Four days later, the bandage was removed; the wound bed revealed highly vascularized granulation tissue. The wound had an oval shape and its size was 4x2x0.5 cm. Three days later, the bandage was removed. The wound had a spindle shape with a size of 3x1.x0.25 cm. The same collagen gel treatment as above was applied. Four days later, the bandage was removed and the size of the wound was found to be 2.5x1x0 cm.

After a few days the patient informed that the wound was completely closed.

EXAMPLE 4

A wound caused by a recurrent crural ulcer was treated.
The ulcer wound (10x7.1x1.5 cm) did not respond to any conventional treatment.

In the first treatment a solution of Example 1 containing 2 ng/ml of human growth hormone and 5 mU/ml (about 200 ng/ml) of Insulin was applied.

A week later, the fibril exudate-stained bandage was removed. A considerable granulation tissue proliferation was noticed that raised the bed of the wound. The size of the wound was found to be 8x5x0.5 cm. The wound was washed with a solution (3% by vol.) of hydrogen peroxide and the same solution as in the first treatment was applied.

Four days later, the bandage was removed and it was noticed that granulation tissue filled most of the gap of the wound which was clean.

A split-skin graft was further applied.

## Examples 5-11

In the following examples 5-11, modified serum-free culture medium was supplemented with non-steroidal anabolic hormones and tested for its wound-healing activity versus numerous controls. The medium was prepared in a purified 1% alginate gel matrix and in 4% gelatin to which physioloigcal concentrations of growth hormone, thyroxin and insulin/transferrin were added.

Under general anaesthesia of Katamin, four 2 X 3 cm full-thickness skin patches were surgically extirpated from the dorsum of Hartley-derived guinea pigs. After application of the gel (about 1cc/cm$^2$) to the wounds, the wounds were dressed with Omiderm, a polyurethane-based synthetic wound dressing (Omikron, Israel) and anchored with gauze and elastic adhesive bandage. Change of the bandages and administration of the gels were performed every 48 hours, under general anaesthesia, at which time in one group, the wounds were washed with ESDC disinfectant (Symbollon Corp., Mass., USA), washed with warm saline, measured and photographed. Computerized morphometric measurements of the photographs were made and the dynamics of the regeneration process were quantified and analyzed. A more detailed description and the results of these experiments is presented herein.

**Materials and Methods**

1. Prepartion of Gel-Media

The whole procedure was performed under sterile conditions.

a. Delivery System

One gram of Agarose Type 1-A; Low EEO (Sigma Chemical Co.) was dissolved in 10 cc of 2X distilled water. The solution was autoclaved. All preparations of the gel media were made using a final concentration of either 1% Agarose or gelatin.

b. Media

The preferred media contained essential and non-essential amino acids, vitamins, other organic constituents, major inorganic salts, trace elements and buffers and was supplemented with CaCl and L-glutamine and with the non-steroidal anabolic hormones, insulin, thyroxin and growth hormone at the concentrations as indicated below.

| Component | Concentration in M |
|---|---|
| **Amino Acids** (L-enantiomers) | |
| Alanine | $1.0 \times 10^{-4}$ |
| Arginine HCl | $1.0 \times 10^{-3}$ |
| Asparagine | $1.0 \times 10^{-4}$ |
| Aspartic Acid | $3.0 \times 10^{-5}$ |
| Cysteine HCl | $2.4 \times 10^{-4}$ |
| Glutamic Acid | $1.0 \times 10^{-4}$ |
| Glutamine | $6.0 \times 10^{-3}$ |
| Glycine | $1.0 \times 10^{-4}$ |
| Histidine HCl | $6.0 \times 10^{-5}$ |
| Isoleucine | $1.5 \times 10^{-5}$ |
| Leucine | $5.0 \times 10^{-4}$ |
| Lysine HCl | $1.0 \times 10^{-4}$ |
| Methionine | $3.0 \times 10^{-5}$ |
| Phenylalanine | $3.0 \times 10^{-5}$ |
| Proline | $3.0 \times 10^{-4}$ |
| Serine | $6.0 \times 10^{-4}$ |
| Threonine | $1.0 \times 10^{-4}$ |
| Tryptophan | $1.5 \times 10^{-5}$ |
| Tyrosine | $1.5 \times 10^{-5}$ |
| Valine | $3.0 \times 10^{-4}$ |
| **Vitamins** | |
| d-Biotin | $6.0 \times 10^{-8}$ |
| Folic Acid | $1.8 \times 10^{-6}$ |
| DL-a-lipoic acid | $1.0 \times 10^{-6}$ |
| Niacinamide | $3.0 \times 10^{-7}$ |
| D-pantothenate 1/20a | $1.0 \times 10^{-6}$ |
| Pyridoxine HCl | $3.0 \times 10^{-7}$ |
| Riboflavin | $1.0 \times 10^{-7}$ |
| Thiamin HCl | $1.0 \times 10^{-6}$ |
| Vitamin B12 | $3.0 \times 10^{-7}$ |
| **Other Organic Constituents** | |
| Acetate | $3.7 \times 10^{-3}$ |
| Adenine | $1.8 \times 10^{-4}$ |
| Choline chloride | $1.0 \times 10^{-4}$ |
| D-glucose | $6.0 \times 10^{-4}$ |
| i-Inositol | $1.0 \times 10^{-4}$ |

(continued)

| | |
|---|---|
| Putrescine 2HCl | $1.0 \times 10^{-6}$ |
| Na Pyruvate | $5.0 \times 10^{-4}$ |
| Thymidine | $3.0 \times 10^{-6}$ |
| **Major Inorganic Salts** | |
| $CaCl_2$ | $4.0 \times 10^{-5}$ |
| KCl | $1.5 \times 10^{-3}$ |
| $MgCl_2$ | $6.0 \times 10^{-4}$ |
| NaCl | $1.2 \times 10^{-1}$ |
| $Na_2HPO_4$ | $2.0 \times 10^{-3}$ |
| **Trace Elements** | |
| $CuSO_4$ | $1.1 \times 10^{-8}$ |
| $FeSO_4$ | $5.0 \times 10^{-6}$ |
| $H_2SeO_3$ | $3.0 \times 10^{-8}$ |
| $MnSO_4$ | $1.0 \times 10^{-9}$ |
| $Na_2SiO_3$ | $5.0 \times 10^{-7}$ |
| $(NH_4)_6Mo_7O_{24}$ | $1.0 \times 10^{-9}$ |
| $NH_4VO_3$ | $5.0 \times 10^{-9}$ |
| $NiCl_2$ | $5.0 \times 10^{-10}$ |
| $SnCl_2$ | $5.0 \times 10^{-10}$ |
| $ZnSO_4$ | $5.0 \times 10^{-7}$ |
| **Buffers** | |
| Hepes | $2.8 \times 10^{-2}$ |
| $NaHCO_3$ | $1.4 \times 10^{-2}$ |
| **Non-Steroidal Anabolic Hormones** | |
| Human Growth Hormone | 2 ng/ml |
| Insulin/Transferrin & sodium selenite | 5 mU/ml (about 200 ng/ml) |
| Triiodothyronine ($T_3$) | $2.0 \times 10^{-9}$ (1.3 ng/ml) |
| **Vehicle** | |
| Agarose (Sigma - A 0169) | 1% |
| EEO (Electroendosmosis 0.10-0.15) | |
| Gel Point - 36°C | |
| Melting Point- 87°C | |
| Gel Strength - > 825 g/cm$^2$ for 1% | |
| pH 7-8.5 | |

### c. Preparation of Wound Healing Formulation

Ninety cc of the above-defined media was warmed in a water bath to 40°C. Following autoclaving, 10 cc of a 1% agarose gel solution was allowed to cool to 40°C and the solution was then added to the media to produce a homogeneous mixture. The mixture was thereafter aspirated into 10 and 20 cc syringes and refrigerated at 4°C.

## 2. Animal Model for Studies

Hartley-derived Albino guinea pigs weighing 300-400 grams were used in this study. The animals were housed in individual cages and fed regularly guinea pig chow and water enriched with Vitamin C ad libitum. All surgical procedures were performed under general anaesthesia using Katamin HCl ([d]-2-(o-chlorophenyl)-2-(methylamino)cyclohexanone hydrochloride, available from Parke-Davis) 150 mg/kg i.m. All histological sections were prepared using Hematoxylene and Eosin stain as well as Mason's trichrome method for collagen.

Each animal was anaesthetized and four bilaterally symmetrical full-thickness skin segments measuring 2 X 3 cm were excised from the dorsum of each animal, two from the scapular region and two from the lumbar region. After washing the wounds with warm saline, the wounds were dressed with the gel at a concentration of about 1 cc/cm$^2$, covered with a polyurethane based synthetic membrane Omiderm (Omikron, Israel) and anchored with gauze, elastic adhesive and a Retelast netting (Medinet, s.p.a., Italy). The dressings were changed every 48 hours under general anaesthesia, at which time the wounds were washed with warm saline to remove any debris and the remaining gel within the wound, measured, photographed and fresh gel was applied and the wounds dressed as described above. In one experiment, a disinfectant compress of ESDC (Symbollon, Corp.) was applied during the change of dressing for about ten minutes, preceded and followed by warm saline rinse. At days 4, 6, 8, 10 and 12, the animals were sacrificed and the wounds with the surrounding tissues were extirpated and prepared for histological examination.

The thickness of the newly formed epithelial layer and of the underlying granulation tissue were measured using light microscopy (Zeiss) at 100x magnification.

The wound macrophotographs were analyzed using ImageMeasure (Phoenix Corp., Seattle, Washington) computerized morphometric program and the experimental results were plotted as graphs showing the fractional change in area (i.e. closure rate) of the wounds treated with gel-media + hormones versus the various controls. The wound closure rate was tabulated and peak closure day (% closure) was determined.

Peak closure rate has been used as a measure of wound healing potency. Peak closure rate is the maximum slope of the

$$\frac{Ao - At}{Ao}$$

vs. time curve. Peak closure rate indicates the time after the start of the treatment at which tissue healing or growth rate reaches a maximum value (maximum rate); i.e., when the treatment is optimal.

### Examples 5-11

Eight groups of experiments were performed using various combinations of medicament as controls:

Experiment 1- Gelatin in saline (n=12) vs. Agarose in saline (n=12).

Experiment 2- Test for Controls- Scarlet Red (n=19) and Agarose in saline (n=12) as positive and negative controls respectively vs. Agarose in hormone supplemented medium with insulin/transferrin (5 mU/ml- about 200ng/ml), Thyroxin (about 1.302 ng/ml) and Human Growth Hormone (about 2ng/ml).

Experiment 3- Agarose in hormone-supplemented medium (n=12) vs. Agarose in medium without hormonal supplement (n=12).

Experiment 4- Agarose with either Insulin/Transferrin (n=33, 5mU/ml- about 200 ng/ml), Thyroxin (n=28, 1.302 ng/ml) or Growth Hormone (n=27, 2 ng/ml) in saline (no medium) vs. medium in Agarose containing all three hormones using the same concentration (n=12) as each of the components specified above.

Experiment 5- Agarose in medium supplemented with the three hormones (n=12) as above vs. Agarose in medium supplemented with either Insulin (n=8), Thyroxin (n=8) or Growth Hormone (n=8).

Experiment 6-Agarose in saline supplemented with the three hormones (n=12) in the concentrations set forth above vs. Agarose in medium supplemented with the three hormones (n=12).

Experiment 7-Agarose in 3 hormone-supplemented medium (as above, n=12) without a disinfectant compress vs. the same medicament plus a 10 minute compress of ESDC disinfectant (Symbollon Corp.) applied during the change of bandages (n=15).

**Example 5-Test for Formulation Delivery Vehicle**

Gelatin and Agarose were prepared in saline and the two gels were used to treat experimental wounds. The rate of closure of the wounds treated with Agarose was faster than the rate of closure of wounds treated with gelatin. (See Figure 1).

Wound closure rate comparison indicates that the closure of 50% was 31% faster with Agarose in Saline as compared to Gelatin in Saline. Peak closure rate occurred 33% earlier with Agarose in Saline treatment. (See Table 1, set forth below).

**Example 6-Test for Positive and Negative Controls**

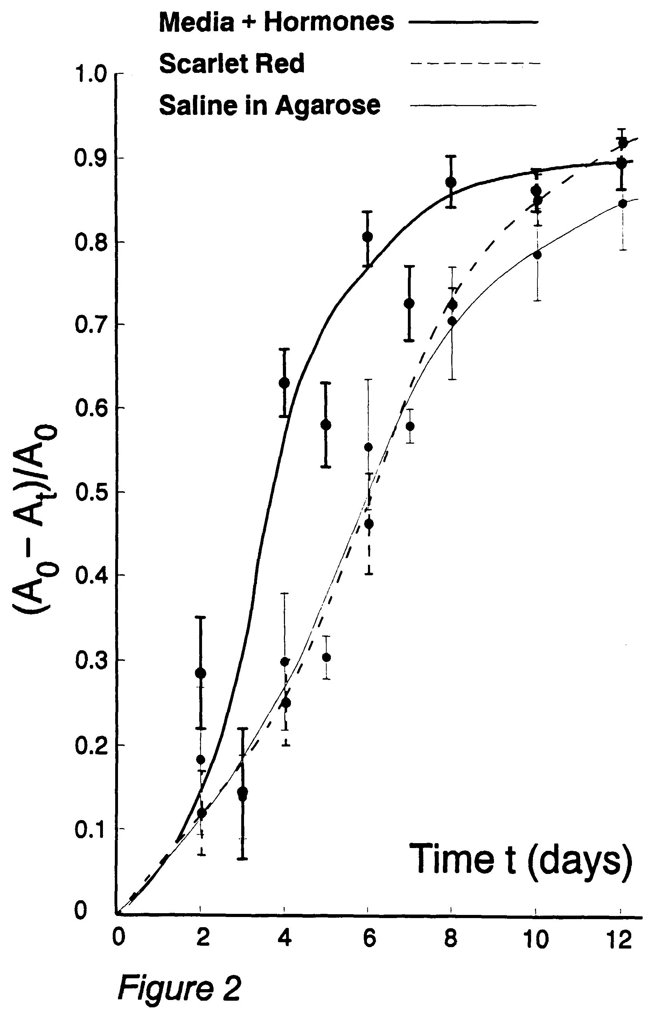

Scarlet red dressing (an azo dye-containing preparation routinely used in hospitals and claimed to increase epitheliazation) was used a positive control and Saline in Agarose was used as a negative control. The rate of wound closure for each of these formulations was plotted against that of the wounds treated with media + hormones (n= 15). Comparison of wound closure rate indicates that 50% closure was 50% faster with media + hormone treatment as compared to both controls. (See Figure 2). Peak closure rate occurred 50% and 40% later for Saline and Scarlet Red treatment, respectively, as compared to media + hormones.

**Example 7- Treatment with Media + Hormones in Agarose vs. Media in Agarose Alone**

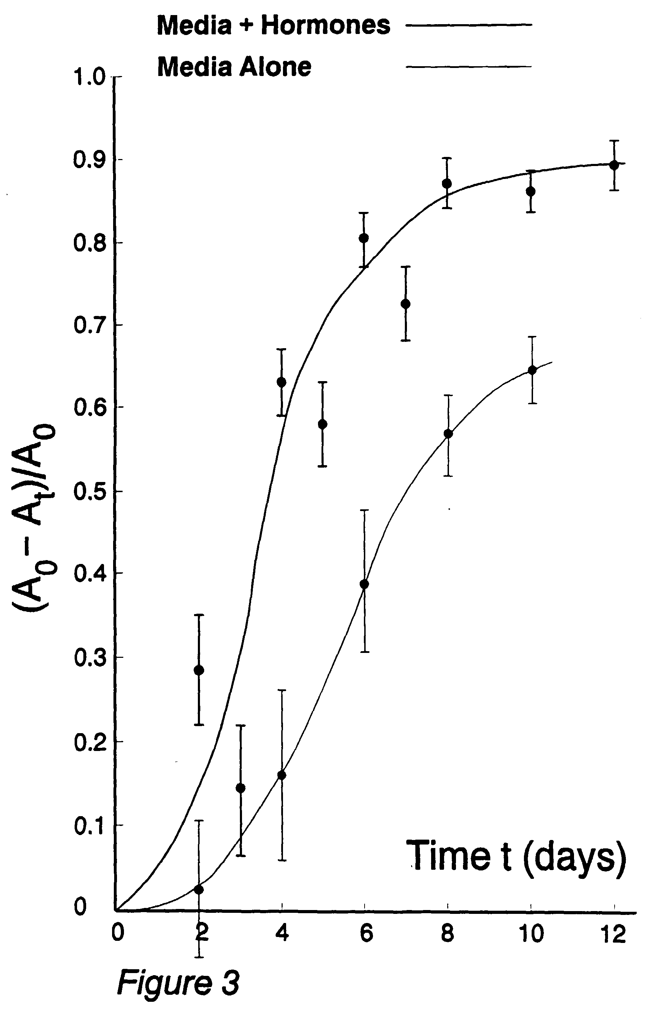

Treatment with the media + 3 hormones formulation induced accelerated wound healing compared to treatment with media prepared in Agarose without hormonal supplement (Figure 3). The rate of wound closure using the media alone was similar to that of the wounds treated with saline in Agarose.

Since media alone did not induce any stimulatory effect on wounds closure, the presence of at least one hormone and preferably three hormones appears essential for utilization of the media by the cells.

Comparison of wound closure rate indicates that closure of 50% was 60% slower with media alone compared to media + 3 hormones (Table 1). Peak closure rate occurred 50% earlier in media + 3 hormone treated wounds compared to wound treated with media alone.

**Example 8- Test of Agarose in Saline (No Medium) Supplemented with either Insulin or Thyroxin or Growth Hormone vs. Treatment with Agarose in Saline Supplemented with the Three**

**Hormones**

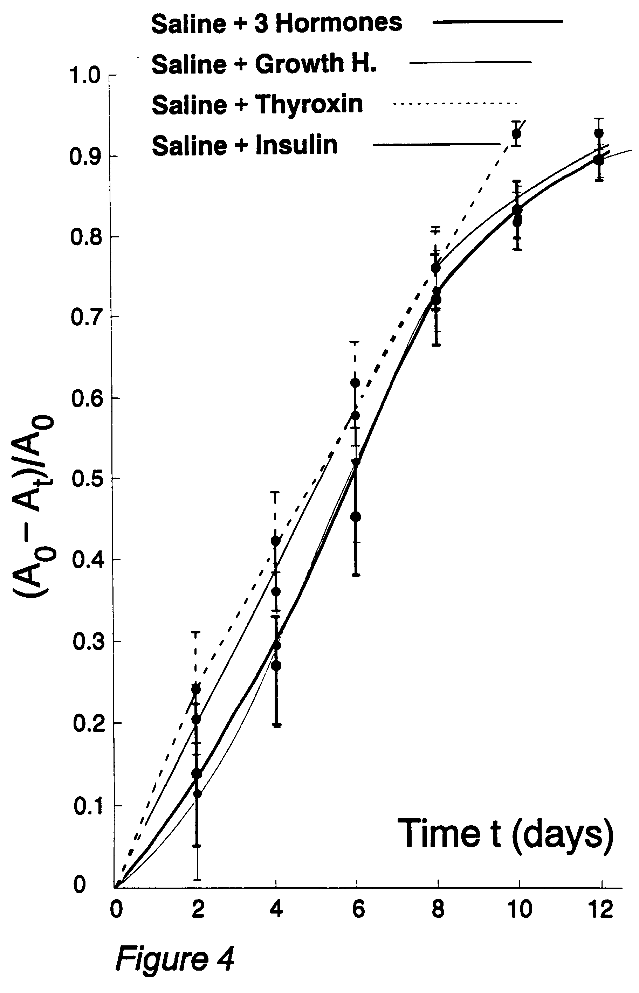

The three hormones together induced a similar rate of growth to treatment with each hormone separately. Thus no synergistic effect was demonstrated by the presence of the three hormones together when mixed with Saline in Agarose (see Figure 4).

Closure rate of wounds treated with each hormone, Insulin, Thyroxin and Growth Hormone separately, were similar. Comparison of closure rates indicated that the closure of 50% was 15% faster for Insulin and Thyroxin and the same for Growth Hormone and Saline + the three hormones together (See table 1).

Peak closure rate was the same for Insulin, Thyroxin and Growth Hormone as well as for saline + 3 hormones.

**Example 9- Test of Agarose in Medium Supplemented with 3 Hormones vs. Agarose in Media Supplemented with either Insulin, Thyroxin or Growth Hormone**

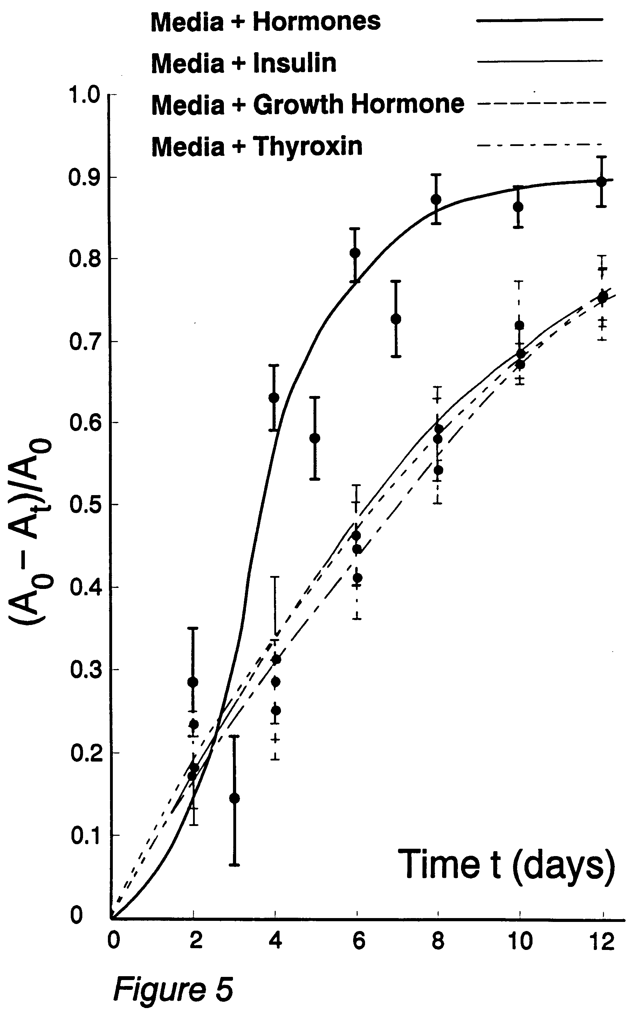

Treatment with Media + 3 hormones yielded significantly faster rate of wound closure (See Figure 5) than Agrose in media supplemented with any one of the three hormones.

Closure rates of wounds treated with media supplemented with one of the hormones, Insulin, Thyroxin and Growth Hormone were similar. However, combination of the three hormones in the media yielded a synergistic effect. Comparison of closure rates indicated that the closure rate of 50% was 75% slower for Insulin, thyroxin and for Growth Hormone compared to the combination of the three hormones. Peak closure rate was the same for Insulin, Thyroxin and Growth Hormone and occurred 33% later than that of Media + 3 hormones together.

**Example 10- Test of Agarose in Saline + 3 Hormones vs. Agarose in Media + 3 Hormones**

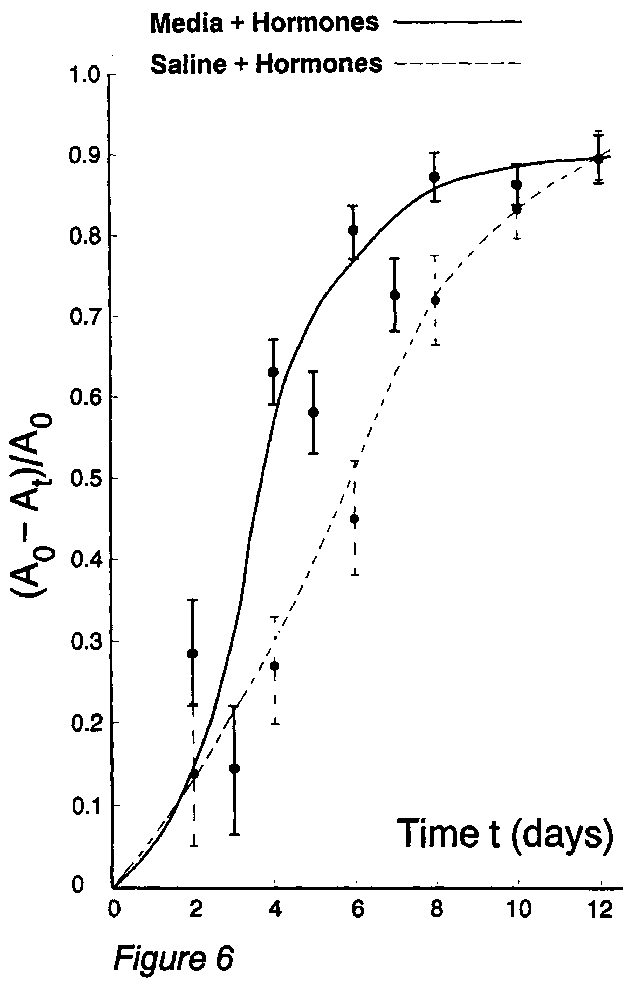

For the first two days the wound closure of both groups was essentially similar. After the second day, however, the rate of wound closure of the group treated with the 3 hormones in saline (without media) was significantly slower than that of the wounds treated with the media + hormones (See Figure 6).

The presence of media induced a faster rate of wound closure as compared to the rate of the same 3 hormones without the media. Comparison of wound clousre rates indicated that the closure of 50% was 86% and slower for the Saline + hormones as compared to the Media + hormones. In addition, peak closure rate occurred 57% earlier in the Media + 3 hormones compared to the Saline + 3 Hormone treatments.

**Example 11- Test of Agarose in Media + 3 Hormones with and without ESDC Disinfectant Compress**

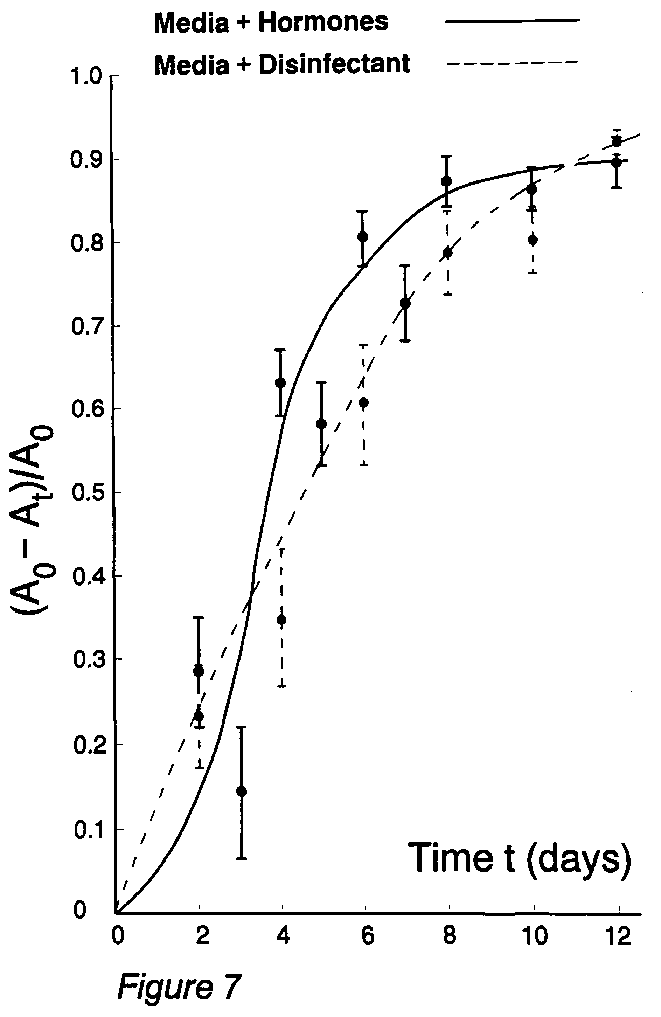

The rate of wound closure with the media + disinfectant treatment was initially faster than that of wounds treated with media alone. However, on the 2nd day the rate of wound closure for wounds treated with media + hormones accelerated and was faster than the closure rate of the wounds treated with the addition of the disinfectant compress. After the initial period of time, the disinfectant exerted a cumulative cytotoxic effect which slowed the healing process. (See Figure 7).

Comparison of wound closure rate indicated that the closure of 50% was 45% slower with the disinfectant treatment.

Peak closure rate occurred 100% later with the disinfectant treatment compared to the medium + 3 hormones without disinfectant treatment.

Table 1

| Wound Closure Rate Comparison | | |
|---|---|---|
| Closure % (Day) | 50% | Peak Closure Day (% Closure) |
| Media + Hormones | 3.5 | 3.0 (30%) |
| Saline in Agarose | 6.0 | 6.0 (50%) |
| Saline in gelatin | 8.5 | 9.0 (50%) |
| Scarlet Red | 6.0 | 5.0 (35%) |
| Media + Disinfectant | 5.0 | 6.0 (60%) |
| Insulin | 5.0 | 3.0 (30%) |
| Thyroxin | 5.0 | 3.0 (30%) |
| Growth Hormone | 6.0 | 6.0 (50%) |
| Media Alone | 7.0 | 6.0 (40%) |
| Saline + Hormones | 7.0 | 7.0 (50%) |

Conclusions

The following conclusions can be drawn from the results of the experiments presented herein:

Drug Delivery System

Using the formulations employed, use of Agarose improves wound closure rate as compared to gelatin. In particular, closure of wounds treated with saline in gelatin is about 33% slower than the closure of wounds treated with saline in Agarose.

Controls- Scarlet Red and Saline

The use of Scarlet Red dressing as positive control and saline as negative control yielded similar and slower closure when compared to media + hormone treatment.

Media and Hormone

The presence of at least one cellular growth stimulating compound in the form of a non-steroidal anabolic hormone and more preferably, a combination of three anabolic hormones in the presence of medium produces significant wound-healing benefit (wound closure rate is significantly higher). Treatment with Gel media (in Agarose) devoid of hormonal supplement was 60% slower than media plus hormone gel (three anabolic hormones) and was similar to the rate of closure found with treatment of Agarose in saline.

Non-Quantifiable observations

The use of media containing hormone in agarose produces a scar which has a soft texture and a smooth surface (an unexpected result). It produces a more aesthetic and natural looking surface area as compared to scarlet red or saline. In most instances, no bulging nor any indentation occurred and the level of the scar tissue is continuous with the conformation of the surrounding non-wounded skin. The texture of the scar is also similar to that of surrounding non-wound tissue and discoloration eventually resolves.

The focus of our interest primarily was the rate of wound closure and our results using the animal model point to the efficacy of media supplemented with cellular growth stimulating compounds according to the present invention, regardless of whether the rate of wound closure was due to wound contraction or epithelialization or to a combination. With these two mechanisms taken into account, the exponential decrease in wound area was nevertheless, significantly faster using media supplemented with hormones. The various controls used in this study illustrate that, both media alone and hormones alone, individually or together did not achieve the closure rates for the wounds treated with both. Furthermore, negative (scarlet red) and positive (saline) controls yielded similar and slower rates of closure.

Since trace quantities of growth factors constitute part of the wound exudate (Freshney, R.I. Culture of animal Cells. Alan R. Liss, Inc., N.Y., 1988, 2nd Edition, pp239-241 and Hayward and Robson, Animal Models of Wound Contraction In: Clinical and Experimental Approaches to Dermal and Epidermal Repair; Normal and Clinical Wounds, pp.301-312, 1991, Wiley-Liss, Inc.), none were added to the formulation of the gel media. While not being limited by way of theory, it is our hypothesis that the application of the gel media into the wound space created a complex, biologically active substrate which may act with the autologous growth factors which, in turn, reinforce the biological activity of the gel. The gel media combines the properties and characteristics of a biologically active material which, in addition, contains all the nutritional requirements for cellular proliferation. Our results regarding the gel's efficacy appear to agree with earlier findings showing early wound exudate to induce cellular proliferation (Mulder, G.D., If wounds could talk. Clinical and Experimental Approaches to Dermal and Epidermal Repair; Normal and Chronic Wounds, pp.55-66, 1991, Wiley-Liss, Inc.

The animal model presented herein imposes certain limitations: wounds are clean, surgically made and uncomplicated by contamination. This point is important since the gel media provides a growth substrate for bacteria. It is believed that, in contaminated wounds, a bacteriogram followed by or concomitant with specific antibiotic or disinfectant treatment in combination with the gel media treatment may be indicated.

This invention has been described in terms of specific embodiments set forth in detail herein, but it should be understood that these are by way of illustration and the invention is not necesarily limited thereto.

**Claims**

1. A formulation for use in the in vivo treatment of wounds in animals and humans comprising wound healing effective amounts of a non-steroidal anabolic hormone selected from the group consisting of insulin, triiodothyronine, thryroxine and mixtures thereof, at a concentration of at least about 0.05 ng/ml in a cellular nutrient medium.

2. The formulation according to claim 1 wherein said nutrient medium is serum-free.

3. The formulation according to claim 1 or 2 wherein said formulation is delivered as a solution, gel or cream.

4. The formulation according to claim 3 wherein said gel is formed by adding an effective amount of a polymer selected from the group consisting of hydroxyethylmethacrylate, polyvinylpyrrolidone, polyethylene glycol, gelatin, agarose, collagen, a hydrophillic cellulose polymer and mixtures thereof.

5. The formulation according to any of claims 1-4 further comprising growth hormone at a concentration of at least 0.5 ng/ml.

6. The formulation according to any of claims 1-5 further comprising hydrocortisone in an amount ranging from 0.1 umol. to 50 umol.

7. The formulation according to any of claims 1-6 wherein said non steroidal anabolic hormone is insulin at a concentration ranging from 5 ng/ml to 100 ug/ml.

8. The formulation according to any of claims 1-7 wherein said nutrient medium is a serum free nutrient medium selected from the group consisting of ADC-1, albumin-free LPM, F10, F12, DCCM1, DCCM2, BGJ Medium, Basal medium Eagle with Earle's salt base, Dulbecco's Modified Eagle Medium, Glasgow Modification Eagle Medium, Leibovitz L-15 Medium, McCoy's 5A Medium, MDCB 153, Medium M199 with Earle's salt base, Medium M199 with Hank's salt base, Minimum Essential Medium Eagle with Earle's salt base, Minimum Essential Medium Eagle with Hank's salt base and Minimum Essential Medium Eagle including non-essential amino acids.

9. The formulation according to any of claims 1-8 wherein said nutrient medium is MDCB 153.

10. The formulation according to any of claims 1-9 further including an effective amount of an antimicrobial agent.

11. The formulation according to claim 1 further comprising human growth hormone.

12. The formulation according to claim 1 further comprising insulin-like growth factor.

**Patentansprüche**

1. Eine Formulierung zur Verwendung bei der in-vivo-Behandlung von Wunden bei Tieren und Menschen, die für die Wundheilung wirksame Mengen eines nicht-steroiden anabolen Hormons umfaßt, das ausgewählt ist aus der Gruppe, die aus Insulin, Triiodthyronin, Thyroxin und Mischungen derselben besteht, in einer Konzentration von wenigstens etwa 0,05 ng/ml in einem Zellnährstoffmedium.

2. Die Formulierung nach Anspruch 1, wobei besagtes Nährstoffmedium serumfrei ist.

3. Die Formulierung nach Anspruch 1 oder 2, wobei besagte Formulierung als eine Lösung, ein Gel oder eine Creme bereitgestellt wird.

4. Die Formulierung nach Anspruch 3, wobei besagtes Gel dadurch gebildet ist, daß eine wirksame Menge eines Polymers zugegeben wird, das ausgewählt ist aus der Gruppe, die aus Hydroxyethylmethacrylat, Polyvinylpyrrolidon, Polyethylenglykol, Gelantine, Agarose, Collagen, einem hydrophilen Cellulosepolymer und Mischungen derselben besteht.

5. Die Formulierung nach einem der Ansprüche 1 bis 4, die außerdem Wachstumshormon in einer Konzentration von wenigstens 0,5 ng/ml umfaßt.

6. Die Formulierung nach einem der Ansprüche 1 bis 5, die außerdem Hydrocortison in einer Menge im Bereich von 0,1 μmol bis 50 μmol umfaßt.

7. Die Formulierung nach einem der Ansprüche 1 bis 6, wobei besagtes nicht-steroides anaboles Hormon Insulin einer Konzentration im Bereich von 5 ng/ml bis 100 μg/ml ist.

8. Die Formulierung nach einem der Ansprüche 1 bis 7, wobei besagtes Nährstoffmedium ein serumfreies Nährstoffmedium ist, das ausgewählt ist aus der Gruppe, bestehend aus ADC-1, albuminfreiem LPM, F10, F12, DCCM1, DCCM2, BGJ Medium, Basal Medium Eagle mit Earle-Salzbasis, Dulbecco's Modified Eagle Medium, Glasgow Modification Eagle Medium, Leibovitz L-15 Medium, McCoy's 5A Medium, MDCB 153, Medium M199 mit Earle-Salzbasis, Medium M199 mit Hank-Salzbasis, Minimum Essential Medium Eagle mit Earle-Salzbasis, Minimum Essential Medium Eagle mit Hank-Salzbasis und Minimum Essential Medium Eagle, einschließlich nicht-essentieller Aminosäuren.

9. Die Formulierung nach einem der Ansprüche 1 bis 8, wobei besagtes Nährstoffmedium MDCB 153 ist.

10. Die Formulierung nach einem der Ansprüche 1 bis 9, die außerdem eine wirksame Menge eines antimikrobiellen Mittels einschließt.

**11.** Die Formulierung nach Anspruch 1, die außerdem menschliches Wachstumshormon einschließt.

**12.** Die Formulierung nach Anspruch 1, die außerdem insulinähnlichen Wachstumsfaktor einschließt.

## Revendications

**1.** Formulation destinée à être utilisée dans le traitement in vivo de lésions chez des animaux et des humains, comprenant des quantités efficaces pour la cicatrisation de lésions d'une hormone anabolisante non stéroïdienne choisie dans l'ensemble constitué d'insuline, de tri-iodothyronine, de thyroxine et de mélanges de celles-ci, à une concentration d'au moins environ 0,05 ng/ml, dans un milieu nutritif cellulaire.

**2.** Formulation selon la revendication 1, dans laquelle ledit milieu nutritif ne contient pas de sérum.

**3.** Formulation selon la revendication 1 ou 2, ladite formulation étant délivrée sous forme d'une solution, d'un gel ou d'une crème.

**4.** Formulation selon la revendication 3, dans laquelle ledit gel est formé par addition d'une quantité efficace d'un polymère choisi dans l'ensemble constitué de méthacrylate d'hydroxyéthyle, de polyvinylpyrrolidone, de polyéthylèneglycol, de gélatine, d'agarose, de collagène, de polymère de cellulose hydrophile et de mélanges de ceux-ci.

**5.** Formulation selon l'une quelconque des revendications 1 à 4, comprenant en outre une hormone de croissance à une concentration d'au moins 0,5 ng/ml.

**6.** Formulation selon l'une quelconque des revendications 1 à 5 comprenant en outre de l'hydrocortisone en une quantité comprise dans l'intervalle allant de 0,1 µmol à 50 µmol.

**7.** Formulation selon l'une quelconque des revendications 1 à 6, dans laquelle ladite hormone anabolisante non stéroïdienne est l'insuline à une concentration comprise dans l'intervalle allant de 5 ng/ml à 100 µg/ml.

**8.** Formulation selon l'une quelconque des revendications 1 à 7, dans laquelle ledit milieu nutritif est un milieu nutritif sans sérum choisi dans l'ensemble constitué de ADC-1, LPM sans albumine, F10, F12, DCCM1, DCCM2, milieu BGJ, milieu basal Eagle avec la base de sel d'Earle, milieu d'Eagle modifié par Dulbecco, milieu d'Eagle modifié par Glasgow, milieu de L-15 de Leibovitz, milieu 5A de McCoy, MDCB 153, milieu M199 avec la base de sel d'Earle, milieu M199 avec là base de sel de Hank, milieu minimum essentiel d'Eagle avec la base sel d'Earle, milieu minimum essentiel d'Eagle avec la base sel de Hank et milieu minimum essentiel d'Eagle comprenant des acides aminés non essentiels.

**9.** Formulation selon l'une quelconque des revendications 1 à 8, dans laquelle ledit milieu nutritif est MDCB 153.

**10.** Formulation selon l'une quelconque des revendications 1 à 9, comprenant en outre une quantité efficace d'un agent antimicrobien.

**11.** Formulation selon la revendication 1, comprenant en outre l'hormone de croissance humaine.

**12.** Formulation selon la revendication 1, comprenant en outre un facteur de croissance de type insuline.

Figure 1

Figure 2

*Figure 3*

*Figure 4*

*Figure 5*

Figure 6

Figure 7